# EUROPEAN PATENT SPECIFICATION

(11) **EP 1 012 148 B1**
(45) Date of publication and mention of the grant of the patent: **15.09.2004**
(21) Application number: 98939787.2
(22) Date of filing: 02.09.1998
(51) Int. Cl.: C07D 319/08, C09K 19/00, G02F 1/13

(54) **MONOACETALS OF BICYCLOHEXYL-4,4-DIONE AND THEIR USE IN PREPARING LIQUID CRYSTALS**
MONOACETALE VON BICYCLOHEXYL-4,4-DIONEN UND IHRE VERWENDUNG IN DER HERSTELLUNG VON FLÜSSIGKRISTALLEN
MONOACETALS DE BICYCLOHEXYL-4,4-DIONE ET UTILISATION DE CES DERNIERS DANS LA PREPARATION DE CRISTAUX LIQUIDES

(30) Priority: 05.09.1997 CH 208297
(43) Date of publication of application: 28.06.2000
(73) Proprietor: Rolic AG, 6301 Zug (CH)
(72) Inventor: BUCHECKER, Richard, CH-8008 Zurich (CH); LUKAC, Teodor, CH-4054 Basel (CH); REICHNARDT, Joachim, D-79639 Grenzach-Wyhlen (DE)
(74) Representative: Liebetanz, Michael, Dipl.-Phys.
(86) International application number: PCT/IB1998/001367
(87) International publication number: WO 1999/012920

(56) References cited:
- EP-A- 0 310 067
- JP-A- 9 194 473
- CHEMICAL ABSTRACTS, vol. 100, no. 25, 18 June 1984 Columbus, Ohio, US; abstract no. 209205y, J.H. BABLER ET AL.: "A facile and efficient method for monoketalization of 1,4-cyclohexanedione" page 542; XP002064266 & SYNTH. COMMUN., vol. 14, no. 1, 1984, pages 39-44,

## Description

The present invention relates to new cyclic monoacetals of bicyclohexyl-4,4'-dione.

Monoacetals of bicyclohexyl-4,4'-dione are important intermediates in the preparation of liquid crystals. Derivatisation of the free keto group in a first step, followed by hydrolysis of the acetal, provides access to a second keto group which then becomes available for a further, different reaction. This step-wise reaction of the monoacetals provides a convenient route for the preparation of a large number of liquid crystal compounds that include a bicyclohexyl, a cyclohexylcyclohexene or a bicyclohexenyl unit in their structure.

The starting material hitherto before used in the preparation of such liquid crystal compounds is 4-(1,4-dioxaspiro[4.5]dec-8-yl)cyclohexanone. This is prepared by acetalisation of bicyclohexyl-4,4'-dione with ethylene glycol. Since the reactivity of the two keto groups of bicyclohexyl-4,4'-dione is the same, a mixture of starting material, monoacetal and diacetal close to the statistical ratio of 1:2:1 is, of course, obtained in the reaction. It is then necessary to isolate the desired monoacetal from the mixture. This involves a relatively complex separation procedure, which means that the theoretical yield of approximately 50 % is further diminished to a significant extent. The reaction of bicyclohexyl-4,4'-dione with diethylene glycol to form 4-(1,4-dioxaspiro[4.5]dec-8-yl)cyclohexanone in accordance with EP 0 310 067, for example, gives a yield of only 34 %. Other references which disclose relatively complex methods of preparing and isolating similar monoacetals include JP 09 194473 A and Chemical Abstracts 100:209205y.

It has been surprisingly found that certain monoacetals of bicyclohexyl-4,4'-dione can be readily separated from both the starting material and from the diacetal.

A first aspect of the present invention thus provides a monoacetal of the general formula I, 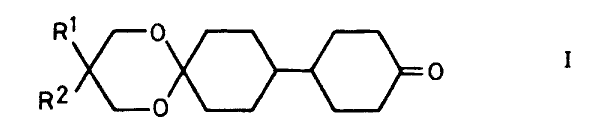 wherein R¹ and R² represent hydrogen or an alkyl group having from 1 to 8 carbon atoms with the proviso that at least one of the substituents R¹ and R² is other than hydrogen.

In formula I of the present invention, the expression "alkyl having from 1 to 8 carbon atoms" includes saturated, unsaturated, branched and straight chain alkyl groups. The alkyl group is preferably a straight chain alkyl group such as *n*-propyl, *n*-butyl, *n*-pentyl, *n*-hexyl, *n*-heptyl or *n*-octyl. Achiral compounds of formula I wherein R¹ and R² represent alkyl and are the same are preferred. Compounds in which the radicals R¹ and R² are the same and represent methyl or ethyl are especially preferred.

The monoacetals of formula (I) have been found to possess physical properties that differ significantly from both the starting material and also from the corresponding diacetals. This means that they can be readily separated from a mixture including the starting materials and the diacetal by the use of very simple steps, e.g. crystallisation. In particular, there is no requirement to use chromatographic methods or the formation of bisulfite derivatives in order to isolate the monoacetal.

The compounds of the general formula I can be prepared in a manner known *per se* by acid-catalysed acetal formation from bicyclohexyl-4,4'-dione and a substituted propane-1.3-diol of the general formula II 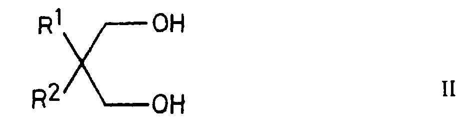 wherein R¹ and R² are as defined above.

A second aspect of the invention therefore provides a method of preparation of a monoacetal of formula (I) which comprises the steps of reacting a bicyclohexyl-4,4'-dione with a substituted propane-1,3-diol of the general formula II as defined above.

The preparation of bicyclohexyl-4,4'-dione is described in the literature, for example in EP 0 310 067. The substance can in addition be obtained commercially.

In addition most of the propane-1,3-diol derivatives of formula II are known compounds. They can be prepared in known manner by mono- or di-alkylation of malonic acid diesters and subsequent reduction of the two ester groups. Some of them are also available commercially.

The monoacetals of the invention can be used for the preparation of liquid crystalline compounds. Such liquid crystalline compounds may be used in the manufacture of optical and electro-optical devices such as TN-cells, STN-cells, TFT-cells or optical components such as refraction gratings, birefringent films and optical retarders. A third aspect of the invention provides the use of a compound of formula (I) in the preparation of a liquid crystalline compound.

The monoacetals of the invention are suitable as intermediates for a broad variety of commercial liquid crystals, which are characterised by the presence of a bicyclohexyl unit in their chemical structure. The synthesis of such liquid crystals is well known and is described in EP 0 310 067 in which 4-(1,4-dioxaspiro[4,5]dec-8-yl)cyclohexanone is used as a starting material. Analogous reactions can be performed with the new intermediates of formula (I) as follows:

The free first ketone group of the compound of formula I is reacted with a first reactive compound followed by deprotecting the second ketone group providing a second intermediate whose remaining ketone group is then allowed to react with a second reactive compound providing the desired mesogenic compound.

Thus Grignard reaction of a suitably substituted phenyl Grignard with the unprotected carbonyl group of the mono-protected bicyclohexyl-4,4'-dione, followed by dehydration, catalytic hydrogenation and hydrolysis of the remaining acetal group, leads to the phenyl substituted bicyclohexyl-4-one intermediate, for example, the 4-fluoro- or the 3,4-difluorophenylbicyclohexyl-4-one derivative. By subsequent Wittig reaction of the liberated ketone with an alkyl-triphenylphosphonium salt followed by catalytic hydrogenation of the double bond, an alkyl side chain of suitable length can be introduced.

The following Example serves to illustrate the invention and does not represent any limitation.

### Example I

First 14.48 g of 2,2-dimethylpropane-1,3-diol and then 0.2 g ofp-toluenesulfonic acid were added at room temperature to a suspension of 38.8 g of bicyclohexyl-4,4'-dione in 400 ml of *n*-heptane. The reaction vessel was then immersed in an oil bath preheated to 120°C and the reaction mixture was stirred at reflux for 25 minutes. 0.5 ml of triethylamine was then added and the mixture was subsequently cooled to 20°C. The suspension obtained in the cooling process was stirred for a further 30 minutes at 20°C and subsequently filtered, and the retained solid material was washed with 250 ml of heptane. The heptane phase was then washed with a 1:1 mixture of water and methanol (twice with 100 ml each time and once with 50 ml) and filtered through sodium sulfate. The filtrate was then concentrated by evaporation, and the residue was dissolved in 125 ml of isopropyl alcohol at 50°C to form a clear solution and subsequently cooled to 23°C. The precipitated crystals, consisting predominantly of diacetal, were then separated off by filtration and the filtrate was concentrated by evaporation, whereupon the residue was dried *in vacuo* at 35°C for 18 hours. This yielded 22.2 g of 4-(3,3-dimethyl-1,5-dioxaspiro[5.5]undec-9-yl)cyclohexanone (corresponding to 39.6 % of the theoretical yield) containing 0.2 % starting material and 1.9 % diacetal. M.p. 81-86°C. (M.p. of the pure compound recrystallised from hexane: 83.7-84.6°C.)

The following compounds can be prepared in an analogous manner:
4-(3,3-diethyl-1,5-dioxaspiro[5.5]undec-9-yl)cyclohexanone;
4-(3,3-dipropyl-1,5-dioxaspiro[5.5]undec-9-yl)cyclohexanone;
4-(3,3-dibutyl-1,5-dioxaspiro[5.5]undec-9-yl)cyclohexanone;
4-(3,3-dipentyl-1,5-dioxaspiro[5.5]undec-9-yl)cyclohexanone;
4-(3-methyl-3-ethyl-1,5-dioxaspiro[5.5]undec-9-yl)cyclohexanone;
4-(3-propyl-1,5-dioxaspiro[5.5]undec-9-yl)cyclohexanone;
4-(3-pentyl-1,5-dioxaspiro[5.5]undec-9-yl)cyclohexanone;
4-(3-isopropyl-1,5-dioxaspiro[5.5]undec-9-yl)cyclohexanone.

## Claims

1. A compound of the general formula I, 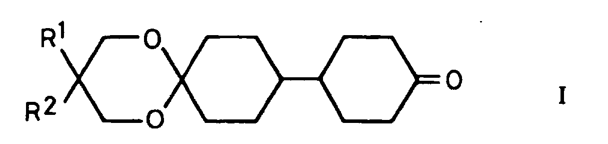 wherein R¹ and R² represent hydrogen or alkyl group having from 1 to 8 carbon atoms with the proviso that at least one of the substituents R¹ and R² is other than hydrogen.

2. A compound according to claim 1, **characterised in that** R¹ and/or R² represent straight-chain *n*-alkyl group.

3. A compound according to claim 1 or claim 2, **characterised in that** R¹ and R² are the same.

4. A compound according to any one of claims 1 to 3, **characterised in that** R¹ and R² represent a methyl or an ethyl group.

5. A method of preparation of a monoacetal of formula (I) according to claim 1 which comprises the steps of reacting bicyclohexyl-4,4'-dione with a substituted propane-1,3-diol of the general formula II 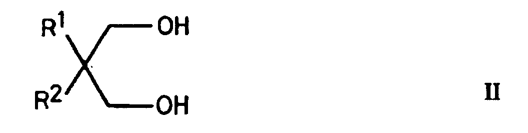 wherein R¹ and R² are as defined above.

6. The use of a compound of formula (I) as defined in any one of claims 1 to 4, in the preparation of a liquid crystalline compound.

7. A method of preparing a liquid crystalline compound, **characterised by** using a compound according to any one of claims 1 to 4.

## Patentansprüche

1. Verbindung der allgemeinen Formel I, 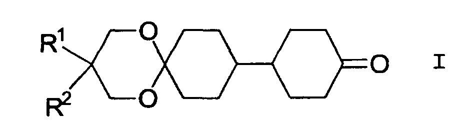 worin R¹ und R² Wasserstoff oder eine Alkylgruppe mit 1-8 Kohlenstoffatomen bedeuten, mit der Massgabe, dass mindestens einer der Substituenten R¹ und R² von Wasserstoff verschieden ist.

2. Verbindung nach Anspruch 1, **dadurch gekennzeichnet, dass** R¹ und/oder R² geradkettiges n-Alkyl bedeuten.

3. Verbindungen nach Anspruch 1 oder 2, **dadurch gekennzeichnet, dass** R¹ und R² gleich sind.

4. Verbindungen nach einem der Ansprüche 1 - 3, **dadurch gekennzeichnet, dass** R¹ und R² eine Methylgruppe oder eine Ethylgruppe bedeuten.

5. Verfahren zur Herstellung eines Monoactetals der Formel (I) nach Anspruch 1, umfassend den Schritt der Reaktion eines Bicyclohexyl-4,4'-dion mit einem substituierten Propan-1,3-diol der allgemeinen Formel II 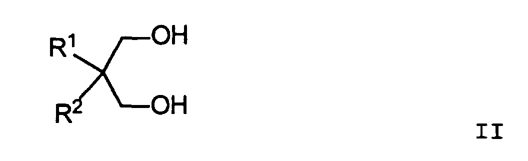 wobei R¹ und R² wie oben definiert sind.

6. Verwendung einer Verbindung der Formel (I) gemäss einem der Ansprüche 1 bis 4 zur Herstellung einer flüssigkristallinen Verbindung.

7. Verfahren zur Herstellung einer flüssigkristallinen Verbindung, **dadurch gekennzeichnet, dass** eine Verbindung gemäss einem der Ansprüche 1 bis 4 verwendet wird.

## Revendications

1. Composé de formule générale I 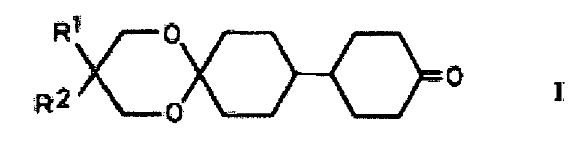 dans laquelle R¹ et R² représentent un hydrogène ou un groupe alkyle ayant de 1 à 8 atomes de carbone avec la condition qu'au moins un des substituants R¹ et R² soit différent de l'hydrogène.

2. Composé selon la revendication 1, **caractérisé en ce que** R¹ et/ou R² représente un groupe n-alkyle à chaîne linéaire.

3. Composé selon la revendication 1 ou la revendication 2, **caractérisé en ce que** R¹ et R² sont identiques.

4. Composé selon l'une quelconque des revendications 1 à 3, **caractérisé en ce que** R¹ et R² représentent un groupe méthyle ou éthyle.

5. Procédé de préparation d'un monoacétal de formule (I) selon la revendication 1 qui comprend les étapes consistant à faire réagir la bicyclohexyl-4,4'-dione avec le propane-1,3-diol substitué de formule générale II 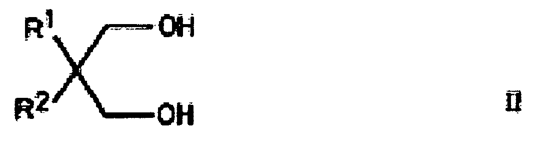 dans laquelle R¹ et R² sont tels que définis ci-dessus.

6. Utilisation d'un composé de formule (I) tel que défini dans l'une quelconque des revendications 1 à 4, dans la préparation d'un composé cristal liquide.

7. Procédé de préparation d'un composé cristal liquide, **caractérisé en ce qu'**on utilise un composé selon l'une quelconque des revendications 1 à 4.
